# EUROPEAN PATENT APPLICATION

(11) **EP 0 535 336 A2**
(43) Date of publication of application: **07.04.1993**
(21) Application number: 92113631.3
(22) Date of filing: 10.08.1992
(51) Int. Cl.: A61K 37/36, C07K 13/00, G01N 33/68

(54) **MK protein as cell growth inhibitor**

(30) Priority: 30.09.1991 US 769029
(71) Applicant: AMERICAN CYANAMID COMPANY, Wayne, NJ 07470-8426 (US)
(72) Inventor: Backer, Joseph Mark, Tenafly, New Jersey 07670 (US); Bohlen, Peter, Peekskill, New York 10566 (US)
(74) Representative: Wächtershäuser, Günter, Prof. Dr.

(57) **Abstract**

The present invention relates to methods for inhibiting cell growth of such cells as endothelial cells by utilizing MK protein.

## Description

The present invention relates to a method for inhibiting cellular growth by the use of the MK proteins.

The protein useful in the present invention is normally produced in the human brain, but apparently at different times developmentally than another heparin binding protein known as heparin binding neurite-outgrowth promoting factor (HBNF) also known as heparin binding neurtrophic factor, and it appears that MK is a member of a highly conserved gene family which is present in a number of different species. The gene encoding the human MK protein has been isolated from a cDNA library obtained from human newborn brain stem RNA and upon expression produces a protein having 121 amino acids.

Unexpectedly, the MK protein has been discovered to be an inhibitor of endothelial responses to basic fibroblast growth factor (bFGF) thereby indicating a potential inhibitory in vivo effect on growth.

### BACKGROUND OF THE INVENTION

In recent years, a number of relatively small polypeptides, known as growth factors, have been identified and isolated. The term "growth factors" refers to a class of signalling substances which affect the growth and differentiation of certain types of animals. This effect can be seen both in the animal and in tissue culture, and a given growth factor may have an effect on more than one type of cell.

Many of the better known growth factors have significant neurotrophic activity, i.e., they are capable of maintaining or stimulating growth of nerve cells. The earliest discovery of such a neurotrophic factor was nerve growth factor (NFG) (Levi-Montalcini and Hamburger, 1953). Similar growth factors which are in the same family as NFG are brain-derived neurotrophic factor (BDNF); (Leibrock et al., 1989) and neurotrophic factor - 3 (NT-3) (Maisonpierre et al., 1990). Additional growth factors include ciliary neurotrophic factor (CNTF) (Lin et al., 1980, IGF-II (Mill et al., 1985), activin (Schubert et al., 1990), purpurin (Berman et al., 1987) and also FGF (Baird and Bohlen, 1990).

A number of known growth factors fall into a superfamily related to fibroblast growth factor (FGF). This family includes basic FGF(bFGF) (Bohlen et al., 1984; Esch et al., 1985), acidic FGF (aFGF) (Bohlen et al. 1985; Gimenez-Gallego et al., 1985) as well as products of the oncogenes int - 2 (Dickens and Peters, 1984), hst/KS (Delli Bovi et al., 1987) FGF-5 (Zhan et al., 1988), FGF-6 (Marics et al., 1989) and KGF (Finch et al., 1989). These are all (except KGF) mitogens for vascular endothelial cells, and all also bind strongly to heparin. Other heparin - binding growth factors, such as VEGF/VPF, are also known (Keck et al., 1989). These heparin-binding growth factors are also frequently isolated from brain tissue and may play a significant role in the growth and development of brain cells.

Kadomatsu et al., 1988) isolated and sequenced cDNA of a retinoic acid-inducible and developmentally regulated gene which was from mouse cells, which protein they referred to as MK. The corresponding mRNA was said to be abundant in the early stages of mouse embryonic development, but not in later stages. The MK1 protein was suggested as being associated with control of cell differentiation, specifically as a DNA binding protein regulating gene expression. No relationship to any other known protein sequences was found. In subsequent papers the expression of the MK gene in early stages of embryonal carcinoma cell differentiation (Tomomura et al., 1990), and the occurrence of three distinct classes of cDNA clones, referred to as MK1, MK2, and MK3 (Kadomatsu et al., 1990) were reported. This suggests that MK may play a fundamental role in the differentiation of a variety of cells, and that it may be involved in the generation of epithelial tissues and in the remodeling of mesoderm.

The mouse MK1 sequence has been shown to have a high degree of homology to the protein known as heparin-binding neurotrophic factors (HBNF); the nucelotide sequence encoding the latter proteins has been disclosed in U.S. patent application Serial No.: 07/568,574. The HBNF proteins were originally disclosed as HBBMs in EP 325076.

The present invention relates to the unexpected discovery that MK inhibits cellular growth.

The proliferation of endothelial cells and subsequent formation of new blood vessels are obligatory steps in the development of several pathological processes including tumor growth, arthritis, and retinopathies (Folkman & Klagsbrun, 1987). Basic fibroblast growth factor (bFGF) is a potent mitogen for endothelial cells in cell culture (Gospodarowicz et. al., 1984) and a powerful angiogenic factor in vivo (Baird & Bohlen, 1990). Endothelial cells express bFGF (Vlodavsky et al., 1987, Moscatelli et al., 1986, Hannan et al., 1987) and can use it as an autocrine growth factor (Sato & Rifkin, 1988). Studies of in vivo and in vitro localization of bFGF indicate that it is also associated with heparin or heparin sulfate moieties in the extracellular matrix and may be liberated by cellular enzymes to activate endothelial cells (Wanka et al., 1991, Vlodavsky et al., 1987, Moscatelli, 1988, Bashkin et al., 1989, and Folkman et al., 1988). Therefore, developing specific and generally non-toxic antagonists of bFGF may be an effective approach to the therapeutic control of angiogenesis.

This strategy to control angiogenesis is particularly effective in view of recent progress in determining the mechanism of interaction between bFGF and cellular receptors. Most cells contain high affinity transmembrane glycoprotein receptors which bind bFGF with a K_{d}=10⁻¹⁰ to 10⁻¹¹M (Moscatelli, 1987). This type of receptor has been identified and several forms of it recently cloned from a variety of species (Ruta et al., 1988, Coughlin et al., 1988, Kornbluth et al., 1988, Lee et al., 1989, Pasquale & Singer, 1989, and Safran et al., 1990) thereby allowing more direct study of the interaction between growth affecting substances and receptors.

In addition to high affinity receptors, cells contain more numerous lower affinity receptors which are heparan sulfate proteoglycans and which bind bFGF with a K_{d}=10⁻⁹M (Moscatelli, 1987). The first member of this type of receptor has been cloned recently (Kiefer et al., 1990). A novel interaction between bFGF binding to low and high affinity receptors was recently reported by Yayon et al. (Yayon et al., 1991). This group showed that proper biosynthesis of cell surface heparan sulfate moieties is necessary for the binding of bFGF to the high affinity receptors. It was also shown that exogenous heparin could restore high affinity binding in cells expressing bFGF receptor but deficient in biosynthesis of heparan sulfates. Yayon et al. suggested that low affinity heparin sulfates proteoglycan receptors bind bFGF, induce conformational change in bFGF, and thus, "create" bFGF capable to bind to high affinity receptors. If the biosynthesis of heparin sulfate proteoglycans is impaired, exogenous heparin or heparin sulfate may induce the proper conformational change in bFGF. This "induced fit" model emphasizes the role of heparin sulfate proteoglycans in the presentation of bFGF to the receptor. In addition it suggests that occupancy of heparin sulfate proteoglycan receptors by heparin-binding proteins may inhibit bFGF binding to high affinity receptor. Indeed, it has been long known that certain heparin-binding proteins are capable of inhibiting angiogenesis and endothelial cell growth in vitro (Taylor & Folkman, 1982, Dauchel et al., 1989). However, the mechanism of action is unknown.

More recently, experimental support for this model was provided by the finding that heparin-binding platelet derived protein PF4 (platelet factor 4) which displays angiostatic activity (Taylor & Folkman, 1982) also inhibits the binding of bFGF to high affinity receptors in NIH 3T3 cells (Sato et al., 1990). Additionally, while PF4 also inhibits both bFGF-driven migration of bovine and human endothelial cells (Sato et al., Sharpe et al., 1990) and bFGF-driven growth of human endothelial cells, it does not affect growth of other normal and tumor cells (Maione et al., 1990, 1991). However, Maione et al. recently presented new evidence which indicates that the mechanism of action of PF4 may not be directly associated with its ability to bind to heparin (Maione et al., 1990, 1991). This group found, that recombinant mutant PF4 and a synthetic C-terminal thirteen-mer fragment of PF4 which both lack affinity to heparin, still retain potent angiostatic activity in vivo, and mutant PF4 inhibits proliferation of human endothelial cells in vitro (Maione et al., 1990, 1991).

MK is structurally unrelated to FGF and PF4 families of growth factors. But it has been discovered that MK proteins displace bFGF from high affinity binding sites on baby hamster kidney (BHK) cells and specifically inhibit growth of bovine and human endothelial cells in vitro, thereby indicating that the MK proteins are cellular growth inhibitors.

### BRIEF DESCRIPTION OF DRAWINGS

Figure 1: Effect of MK on bFGF binding to BHK cell high affinity receptors. - Assay is described in Example 8.
Figure 2: Effect of MK on bovine ABAE cell growth. Assay is described in Example 6. Panel A: MK inhibits basal (●) and bFGF-stimulated (▲) (1ng/ml) ABAE cell growth with ED₅₀=0.04µM. Panel B: 0.37µM MK do not cause detachment of ABAE cells. Subconfluent ABAE cells are exposed to 0.37µM MK for 4 days, during which no visible detachment of cell occurs. "Start"-refers to plates prior to the addition of MK; "Control"-refers to plates which are not treated with MK; "MK"-refers to plates treated with MK. Assays are in duplicates.
Figure 3: Effect of MK on human HUVE cell growth ●-1 ng/ml bFGF in media; ▲ -10ng/ml bFGF im media. Assay is described in Example 9.
Figure 4: Effect of 0.75µM MK on human forskin fibroblasts growth. Open bars -no bFGF, closed bars -10 ng/ml bFGF in media. "Control" -refers to untreated cells; "MK" - refers to cells treated with 0.75µM MK for 4 days. Assay is described in Example 9.
Figure 5: Shows a nucleotide sequence and deduced amino acid sequence of human and mouse MK. Differences in the two nucleotide sequences are indicated by stars (*). Differences in the amino acids are indicated in bold letters. Amino acids used in the mouse genomic PCR primer design are underlined.

### SUMMARY OF THE INVENTION

The present invention relates to a method to inhibit cellular growth by administering MK to an animal preferably a warm-blooded animal. One type of cellular inhibition of tremendous consequence is inhibition of angiogenesis, the vasularization or formation of new blood vessels. Inhibiting vascularization in diseases such as solid tumors, rheumatoid arthritis and eye diseases such as retinopathies, neovascular glaucoma, ocular tumors and the like is important in the medical treatment of not only human beings but may be useful in veterinary therapies, as well. In addition to treating disease states, the control of post-surgical bleeding in by-pass surgery, for instance, is also provided with the compounds of the present invention. Additionally, the use of the present invention can drive cells into a more differentiated state, thereby providing a chemotherapeutic use, as well. This may be achieved by direct administration of the purified protein or may also be achieved by transplant of transgenic host cells capable of producing the protein into the region the body seeding such treatment. Furthermore, angiogenesis or the formation of new capillaries, is critical in a variety of pathological states or diseases. For instance, the following are but a few of the angiogenesis-dependent diseases: angiofibroma, arterinvenous malformations, arthritis, artherosclerotic plaques, corneal graft neovascularization, delayed wound healing, diabetic retinopathy, granulations-burns, hemangioma, hemophilic joints, hypertrophy scars, neovascular glaucoma, nonunion fractures, Osler-Weber Syndrome, psoriosis, pyogenic granuloma, retrolental fibroplasia, scleroderma, solid tumors, trachoma and vascular adhesions (Moses, et al., 1991).

In addition to the above-discussed in vivo therapies, the compounds useful in the present invention provide another use, namely in vitro screening mechanism to identify FGF agonists and antagonists.

Both native sources and recombinantly-derived MK are contemplated for use in the present invention as well as analogues of MK. For instance, alkylated analogues such as carboxymethylated are useful in the present invention.

Thus, the objectives of the present invention relate to providing in vivo inhibitors of cell growth and in vitro assays or screening methods and to therapeutic methods which comprise administering effective amounts of MK in vivo to an individual in need of such or using MK in an endothelial cell assay. These and other objectives of the present invention are more apparent by the detailed description provided hereinbelow.

The following examples are presented for purposes of illustration only and are not to be considered as limiting the scope of the present invention.

### DETAILED DESCRIPTION OF THE INVENTION

### Example 1

The DNA sequence encoding human MK is cloned by a combination of polymerase chain reaction (PCR) and screening of a cDNA library derived from newborn human brain stem. The human HBNF sequence is used as the starting point for designing oligonucleotides for a PCR amplification reaction. Specific oligonucleotides are designed to the regions most conserved between HBNF and the published mouse MK1 DNA sequence. These oligonucleotides are used as primers in a polymerase chain reaction (PCR) on mouse genomic DNA. The expected 150 base pair product is cloned in an appropriate vector and the sequence determined. This clone is used as a probe for screening a human brain cDNA library to identify the human MK equivalent gene. A single clone is isolated, subcloned and sequenced. The nucelotide sequence is subsequently confirmed in additional shorter-length MK clones, which are found to contain different overlapping fragments of the original clone. The sequence of the MK cDNA includes two polyadenylation signals and a poly A tail. The original isolated clone has an open reading frame with a coding region beginning at nucleotide 22 and defining a 143 residue protein. The N-terminal sequence is highly hydrophobic and has the characteristics of a signal peptide (Von Heijne, 1985). On the basis of the criteria for signal peptide structures set forth by Von Heijne id. and Von Heijne, 1986) and comparisons with mouse MK and human HBNF sequences, it is assumed that signal peptide cleavage occurs between amino acid residues 22 (Ala) and 23 (Lys), thus giving rise to a mature MK polypeptide of 121 residues in length.

To provide a source of the mature MK protein free of MK protein free of contaminating eukaryotic proteins, cDNA clones isolated above are used as templates for PCR amplification with primers designed to place a methionine codon immediately 5' of the N-terminal lysine residue of the mature proteins. The amplified product is cloned into a modified form of the expression vector pET-3a (Studier, et al., 1990), and the resulting plasmid pETMH2 is transformed into E. coli strain BL21 LysS. Protein extracts of IPTG-induced pETMH2-containing bacteria express a major protein band migrating at approximately 16.5 kDa. Uninduced culture contains much less of the protein as judged by SDS-PAGE band intensities. Recombinant MK protein is purified from IPTG-induced bacterial cultures by heparin affinity chromatography, mono-S cationic exchange and/or reverse phase HPLC, and its N-terminal sequence and amino acid compositions confirmed.

Homology between the human and published mouse MK DNA and the deduced protein sequences show a lower level of conservation than a similar evolutionary comparison of HBNF. Using a putative N-terminus from the mature MK protein deduced from homology with HBNF, 86% amino acid identity is observed including a three amino acid deletion in the mouse sequence. Both HBNF and MK are expressed in brain but their temporal and spatial regulation differs. Preliminary in situ hybridization show distinct patterns of expression for the two messages. These initial experiments indicated that MK is not expressed in any adult tissue examined while HBNF mRNA is prominantly expressed in adult brain. However, subsequent experiments indicate that MK mRNA is detectable in two regions of the adult brain, the caudate nucleus and the brain stem. Based on the significantly longer exposure times needed to see these bands in adult RNA as compared to equivalent amounts of embryonic RNA, it appears that MK RNA is expressed at minimal levels in the adult.

The temporal expression of MK and HBNF genes is evaluated by northern blot analysis with total rat RNA from various developmental stages. Hybridization with an HBNF probe indicates a gradual increase of message throughout development, with the highest level occurring in the adult brain. Hybridization of the same blot with an MK probe indicates that only 12-, 14- and 16-day embryonic tissues contain the message. The most abundant presence of MK message appears to be in the embryonic day 12 stage. These results are in general agreement with in situ hybridization studies of Kadomatsu (supra). However, contrary to the findings of Kadomatsu, MK mRNA expression in adult kidney tissue is not detected. Studies of HBNF protein in rat brains suggest that the highest level occurs in postnatal day-7 pups. This level reflects a ten-fold difference when compared to 56-day old animals (Rauvala, supra).

The human embryonal carcinoma (EC) cell line NT2/D1 can be induced to differentiate at concentrations of retinoic acid (RA) varying from 0.01 to 10 µM, with the proportion of differentiating EC cells ranging from 50% at 0.01 µg RA to greater than 99% at 1 and 10 µgM RA. The expression of MK and HBNF during differentiation of NT2/D1 is studied, at concentrations ranging from 0.01 to 10 µM. After nine days of exposure to RA, total RNA was extracted from cells and probed for gene expression by northern analysis. Expression of both gene follows a similar pattern. Levels of mRNA expression remain at a steady background level. When RNA hybridization signals are normalized to a control β-actin probe, the maximum increases are calculated to be 6-fold for HBNF and 11-fold for MK. These results are comparable to those observed for MK during retinoic acid induction of the mouse EC cell line, HM-1 (Kadomatsu et al., supra). In this cell line, MK gene expression is induced 8-10 fold above background.

Thus HBNF and MK seem to be members of a highly conserved gene family. Furthermore, the gene expression data implies that these genes may function in the proliferation, maintenance and/or developmental differentiation of tissue and, in particular, nerve tissue.

The following illustrates the cloning and expression of the MK gene in a T7 RNA polymerase expression system. However, although this T7 expression system is quite efficient, it is to be understood that this is not the only means by which MK can be produced recombinantly. Production of MK can be achieved by incorporation of the MK gene into any suitable expression vector and subsequent transformation of an appropriate host cell with the vector; alternately the transformation of the host cells can be achieved directly by naked DNA without the use of a vector. Production of MK by either eukaryotic cells or prokaryotic cells is contemplated by the present invention. Examples of suitable eukaryotic cells include mammalian cells, plant cells, yeast cells and insect cells. Similarly, suitable prokaryotic hosts, in addition to E. coli, include Bacillus subtilis.

Other suitable expression vectors may also be employed and are selected based upon the choice of host cell. For example, numerous vectors suitable for use in transforming bacterial cells are well known. For example, plasmids and bacteriophages, such as λ phage, are the most commonly used vectors for bacterial hosts, and for E. coli in particular. In both mammalian and insect cells, virus vectors are frequently used to obtain expression of exogenous DNA. In particular mammalian cells are commonly transformed with SV40 or polyoma virus; and insect cells in culture may be transformed with baculovirus expression vectors. Yeast vector systems include yeast centromere plasmids, yeast episomal plasmids and yeast integrating plasmids.

It will also be understood that the practice of the invention is not limited to the use of the exact amino acid sequence of the MK protein. Modifications to the DNA sequence of the MK protein such as deletions, insertions, or substitutions in the sequence which produce silent changes in the resulting protein molecule are also contemplated. Also, modifications to the amino acid sequence which result in biologically active MK proteins are included within the scope of the present invention.

For example, alterations in the gene sequence which result in the production of a chemically equivalent amino acid at a given site are contemplated; thus, a codon for the amino acid alanine, a hydrophobic amino acid, can readily be substituted by a codon encoding another hydrophobic residue, such as glycine, or may be substituted with a more hydrophobic residue such as valine, leucine, or isoleucine. Similarly, changes which result in substitution of one negatively charged residue for another, such as aspartic acid for glutamic acid, or one positively charged residue for another, such as lysine for arginine, can also be expected to produce a biologically equivalent product. Nucleotide changes which result in alteration of the N-terminal and C-terminal portions of the protein molecule frequently do not alter protein activity, as these regions are usually not involved in biological activity. It may also be desirable to change a hydrophobic amino acid to a charged amino acid if no adverse effect is observed in biological activity. Each of the proposed modifications is well within the routine skill in the art, as is determination of retention of biological activity of the encoded products. Therefore, where the phrase "MK" or MK protein" is used either in the specification or the claims, it will be understood to encompass all such modifications and variations which result in the production of biologically equivalent MK protein.

The MK protein is strongly homologous to the HBNF protein, and like HBNF stimulates induction of neurite outgrowth. MK is therefore proposed as a neurotrophic agent. As such, use of the MK protein for both in vivo and in vitro for the growth, maintenance and repair of nerve cells of the peripheral and central nervous systems is possible. An example of in vitro application is in maintenance of embryonic brain implants which are now proposed for use in treatment of Parkinson's disease.

In view of the apparent role in differentiation, the MK protein is also proposed as a tissue differentiation, maintenance and repair factor. In particular, MK may be useful in treatment of tumor cells to induce reversion to a differentiated phenotype.

The identification of the gene and its sequence permit construction of transgenic cells such as fibroblasts, monocytes, or macrophages, which may be engineered to permit expression of the MK gene and used as an implant for treatment of the diseases previously discussed.

Moreover, the therapeutic use of MK is not limited to treatment of humans beings alone. In fact, in view of the conserved nature of this protein among distantly related species, administration of MK in any form may be beneficial for veterinary application as well. Therapeutic compositions comprise MK in an amount effective to induce the desired biological activity in combination with a pharmaceutically acceptable liquid or solid carrier. Alternately, the composition comprises a pharmaceutically acceptable aggregation of compatible transgenic cells capable of expressing MK in vitro, as an implant for peripheral and central nervous system repairs or differentiation treatment.

### Example 2

### Cloning and Sequencing of the MK Gene

The published mouse MK protein amino acid sequence is used to create specific oligonucleotides to be used as primers in a polymerase chain reaction. Mouse genomic DNA was isolated from C57 Black/6J mice, as described in Maniatis et al. supra.

A sense primer is made to the amino acid sequence: Cys-Asn-Trp-Lys-Lys-Glu-Phe-Gly starting with a HindIII restriction site and comprised of the DNA sequence: 5'-CAAGCTTGCAACTGGAAGAAGGAATTTGAA-3'. An antisense primer is made to the amino acid sequence Asn-Ala-Gln-Cys-Gln-Glu-Thr starting with the EcoR1 site and comprised of the DNA sequence: 5'GGAATTCGGTCTCCTGGCACTGGGCAGT-3'.

The PCR reaction is carried out on the complementary DNA template with a minute annealing at 50^{o}C, 2 minutes extension at 72^{o}C and 1 minute denaturation at 94^{o}C for 30 cycles using Taq polymerase (USB Corp.).

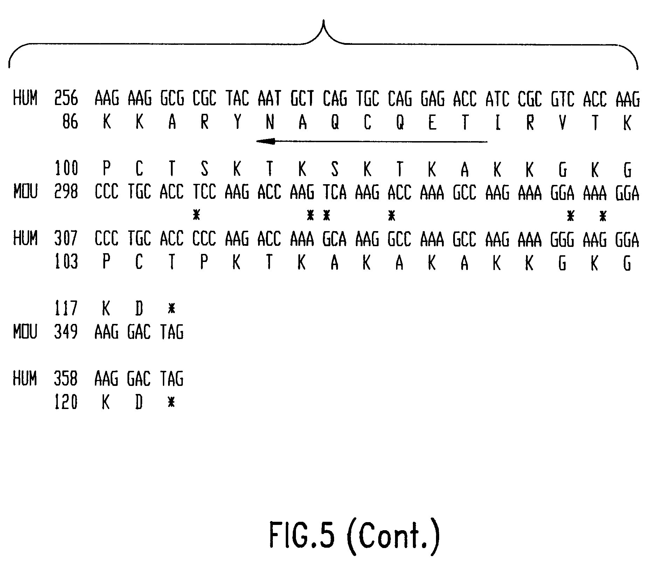

The 150 base pair mouse MK PCR product is cloned into Blue Scribe (+) vector (Stratagene) and used as a probe in screening a newborn brain stem and basal ganglia λ gt 11 cDNA library. A single putative clone containing the MK sequence is isolated and subcloned into the EcoRI site of Blue Scribe (+) and sequenced by the dideoxynucleotide chain termination method. The sequence of the MK gene, as well as the predicted amino acid sequence is presented in Figure 5. Comparison with the mouse MK sequence shows a 41 nucleotide difference, including the three codon deletion in the mouse sequence.

### Example 3

### Expression of Recombinant Human MK

The isolated clone noted above, referred to as pMKHC2 is used as a template for PCR amplification with primers designed to place a methionine codon and an Nde I restriction site immediately 5' to the N-terminal lysine. The purified PCR product is cloned into a derivative of the expression vector pET-3a, which is modified by the deletion of the 1400 bp Sa11/PvuII fragment and insertion of an f1 origion of replication into the EcoRI site. After sequencing the insert to confirm the fidelity of the PCR amplification, the plasmid (named pETMH2; also previously referred to as pETMKHC2) is transformed into strain GL21 LysS and induced for protein production with IPTG as described (Studier et al., supra). Pellets from one ml culture are resuspended in 100 µl of SDS buffer (Laemmli, 1970) and 2.5 µl run on a 15 acrylamide SDS-PAGE gel. The gel is stained with coomassie blue. Recombinant MK is purified from bacterial extract on heparin sepharose CL-6B (Pharmacia) resin in 10 mM Tris, pH 7.0 and eluted at 1-1.13 M NaCl. Further purification is achieved on Mono S (Pharmacia) columns in 50 mM sodium phosphate, pH 6.8, with increasing salt concentration from 0 to 1 M NaCl. Purified protein is eluted at .6 M NaCl.

### Example 4

### Neurite Outgrowth Assays

Brains from 18-day fetal rats are removed under sterile conditions and dispersed to single cells in DMEM containing 10% FCS using a sterile 5 ml syringe. The cell suspension as adjusted to 5 x 10⁵ cells/ml and plated onto tissue culture dishes that are precoated with 50µg/ml poly-L-lysine for 30 minutes at room temperature (Rauvala and Pihlaskari, 1987). Cultures are incubated for 24 hours at 37^{o}C in 10% Co₂, after which the media are changed to DMEM containing 1 mg/ml BSA, and HBNF or MK proteins are added at indicated concentrations. After a further one-day incubation, neurite outgrowth activity is determined by visual examination of cells for extended outgrowth/processes as compared to controls. As shown in Figure 5D, purified recombinant MK is capable of stimulating neurite outgrowth to substantially the same extent as recombinant HBNF and bovine brain derived HBNF.

### Example 5

### Growth and Retinoic Acid Induction of the Human NT2/D1 Cells

The human embryonal carcinoma cell line Nt2/D1 is grown. For retinoic acid induction, cells are grown and resuspended in DMEM medium containing 10% bovine calf serum and resuspended in DMEM medium containing 10% bovine calf serum (Hyclone Laboratories, Inc.) at a density of 5 x 10⁵ cells per 100 mm dish. Varying concentrations of all-trans retinoic acid in dimethyl sulfoxide (10 µl) is added and cells are incubated for 9 days. Fresh medium and RA are added at days 4 and 8. Plates are washed once with phosphate buffered saline, and RNA extracted as described above. Since NT2/D1 cells induced with RA have been suggested as providing a model system for studies of neuronal differentiation, the increase in induction of HBNF and MK genes in this system indicates a possible role in neuronal cell development.

### Example 6

### Other Proteins

Human, platelet-derived PF4 is purchased from Sigma in St. Louis. Recombined bFGF and analogues with wildtype activity and potency are expressed and purified as described in (Seddon et al., 1991). ¹²⁵I-bFGF (1,000Ci/mmole) is purchased from Amersham.

Carboxymethylated MK is prepared as follows: Lyophilized recombinant MK is dissolved in 0.1M Tris-HC1 pH 8.6, containing 2mM EDTA and 4.5M guanidinium HC1 to give a concentration of 0.5 mg/ml. The protein is reduced with dithiothreiotol (5mM) and the solution incubated under an argon atmosphere for 1 hour at 37^{o}C. The reduced protein solution is cooled to room temperature and alkylated using iodoacetic acid (15mM) for 1 hour in the dark. The carboxymethylated protein is dialysed (35000 molecular weight cut-off) overnight at 4^{o}C versus 10mM Tris-HC1 pH 7.2 containing 200mM NaC1. Carboxymethylcysteine and protein concentrations are determined by amino acid analysis after HC1 gas phase hydrolysis (5.7M HC1/01.%phenol;24h at 100^{o}C) using a model 420A PITC-derivatizer equipped with an on-line model 130A separation system (Applied Biosystems, CA). Carboxymethylated MK is eluted from Heparin-sepharose with 0.9M NaC1, while native MK is eluted with 1.1M NaCl

### Example 7

### Cells Lines

BHK cells are grown in Dulbecco's Modified Eagle Medium (DMEM) (Mediatech, Washington, D.C.) supplemented with 7.5% fetal calf serum (Gibco). Human foreskins fibroblasts (HFSF) and human melanoma cell lines, gifts from Dr. Eisinger (Lederle Laboratories, Pearl River, NY) are grown in DMEM media (Mediatech, Washington, D.C.) supplemented with 5.0% fetal calf serum (Gibco). Bovine ABAE cells are grown in DMEM supplemented with 10% calf serum (Hy Clone). Human umbilical vein endothelial (HUVE) cells (passage 1), a gift from Dr. Jaffe (Cornell University, Medical Center, New York, NY) are grown in Media 199 (Mediatech, Washington, D.C.) supplemented with 4% serum (Gibco) and 16% fetal calf serum (Gibco) as described by Jaffe (1984).

### Example 8

### Radioreceptor Assay

¹²⁵I-bFGF binding to high affinity receptors on BHK cells is performed according to Moscatelli (1987). Briefly, cells are incubated with 50pM of ¹²⁵I-bFGF and various additions for 1 hour at room temperature, then incubated at 4^{o}C for 30 minutes. High salt (2M) washing of the cells releases bFGF from low affinity sites, while treatment of the cells with 0.5% Triton X100 releases bFGF from high affinity sites. The assay is validated by a displacement experiment in which increasing concentrations of non-radioactive bFGF are added to the incubation mixture. Non-radioactive bFGF decreases the amount of radioactivity released by Triton X100 in a dose-dependent manner with an ED₅₀ of 50pM. Binding assays are done in duplicates.

### Example 9

### Mitogenic Assays

Mitogenic assays are performed according to Fafeur et al. (1990). Briefly, bovine ABAE cells are plated in multi-well dishes at 8,000 cells/well with or without 1ng/ml bFGF for 4 hours, followed by addition of increasing concentrations of heparin-binding proteins. In the presence of 1ng/ml of bFGF the number of ABAE cells is 3-4 fold higher than in the absence of bFGF. HUVE cells are seeded in gelatin coated 24-well plates at 8,000 cells/well and compounds are added 16 hours after seeding. After four days, cells are detached and counted. Mitogenic assays are done in duplicates.

### Example 10

### Results of Assays and Binding Studies

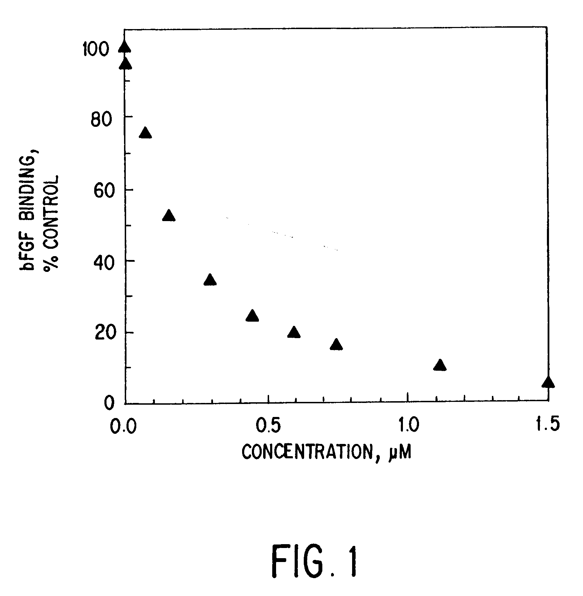

The effects of MK on bFGF binding to high affinity receptors of BHK cells disclose that MK inhibits bFGF binding with an ED₅₀ of 0.1µM (Figure 1). This inhibitory activity of MK is destroyed by reducing disulphide bonds in MK with DTT (dithiothreitol). This treatment presumably prevents proper folding of MK and slightly decreases the MK affinity for heparin-sepharose.

Like MK, PF4 inhibits bFGF binding in a dose-dependent manner with an ED₅₀ of 0.25µM. The latter value is identical to that deduced from the data obtained in similar experiments with NIH 3T3 cells (Sato et al., 1990).

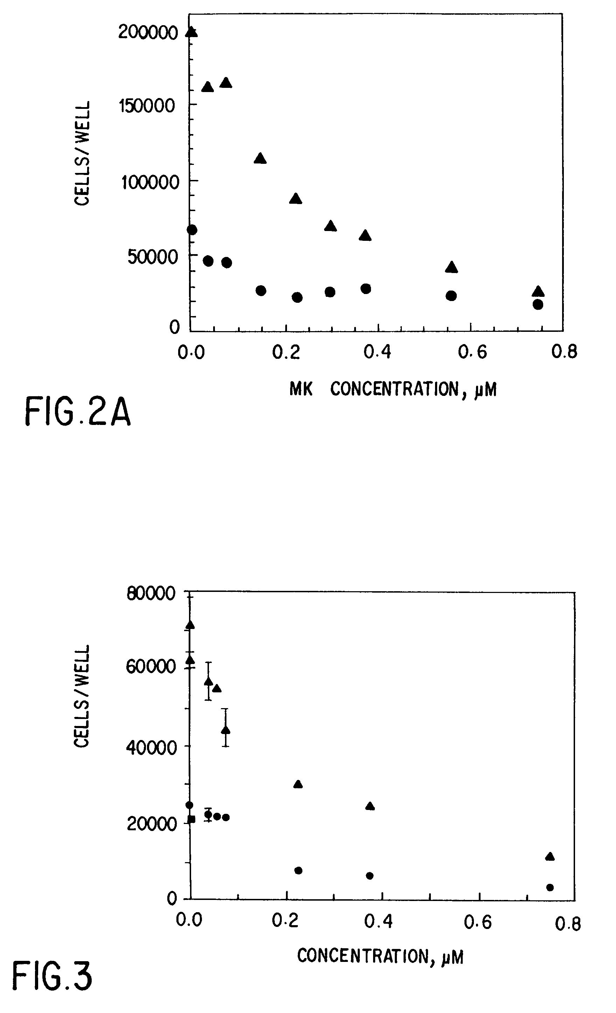

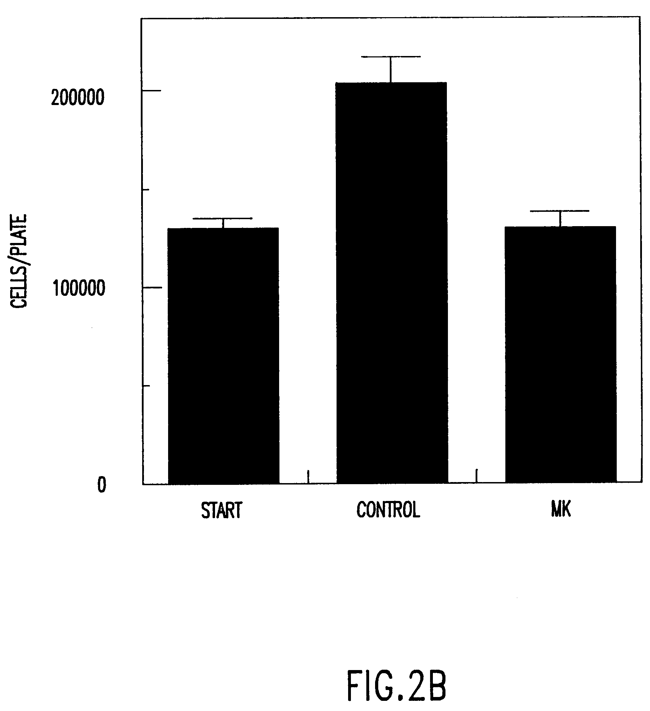

MK inhibits basal and bFGF-stimulated growth of ABAE cells in a dose-dependent manner with an ED₅₀ two-fold higher in the presence of bFGF (∼0.08µM without bFGF, and -0.18µM with bFGF, Figure 2A). Growth of cells is almost completely inhibited at concentrations of MK above 0.2µM. However, the number of cells remains constant and no detached cells appear with doses of 0.37µM MK (Figure 2B). Human PF4 also inhibits bFGF-driving ABAE cell growth in a dose-dependent manner with an ED₅₀ of 0.04µM. High concentrations of PF4 not only inhibit growth of ABAE cells but also cause visible detachment of ABAE cells from the plate.

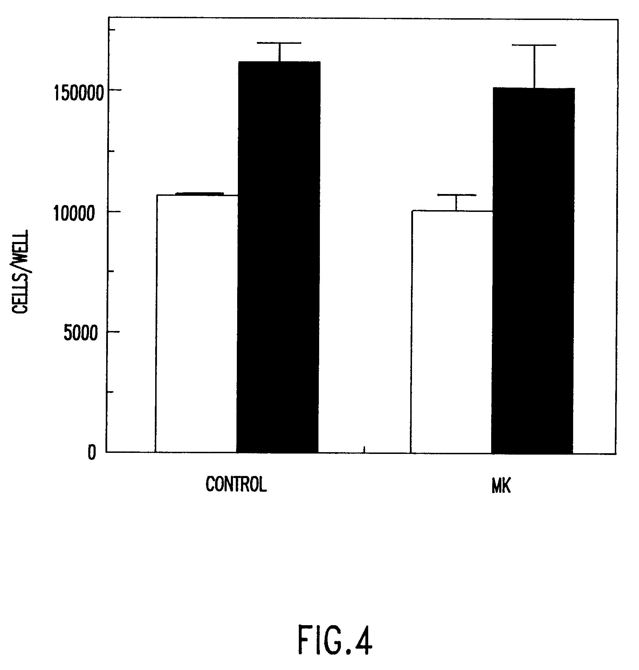

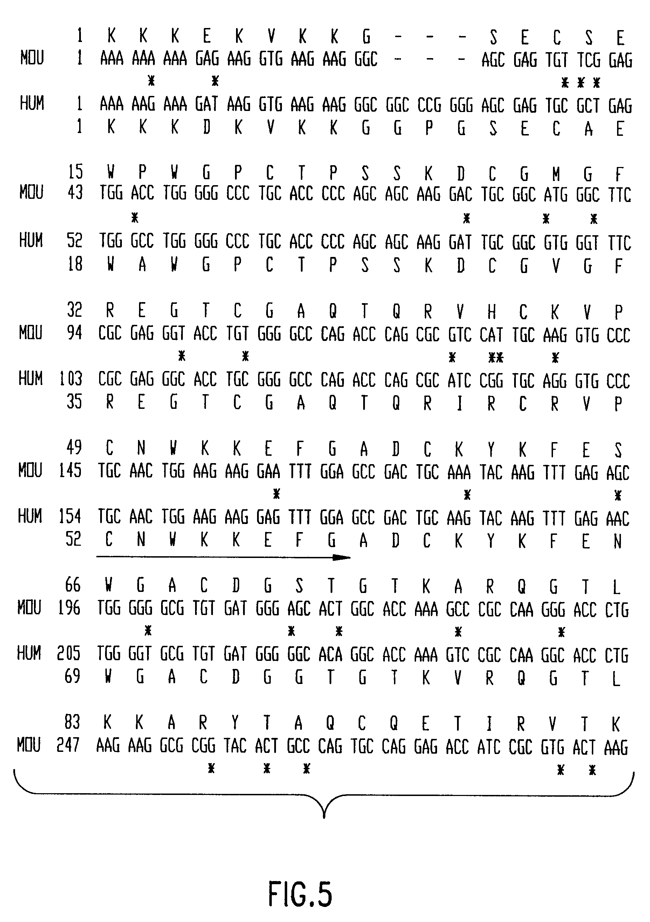

MK also inhibits bFGF-stimulated growth of human primary endothelial cells (HUVE cells) at bFGF concentrations of 1 ng/ml and 10 ng/ml. The bFGF-driven growth of HUVE cells is inhibited by MK in a dose-dependent manner at both concentrations of bFGF with similar ED₅₀ values. High concentrations of MK (above 0.2µM) inhibit HUVE cell growth to a lesser extent at 10 ng/ml bFGF than at 1 ng/ml bFGF (Figure 3).

An anti-proliferatory activity of MK is not observed with hamster BHK cells and rat PC12 cells. These proteins also do not affect growth of two non-endothelial human cell lines: normal human foreskin fibroblasts (HFSF) and a highly malignant melanoma cell line. With both cell lines, 0.75µM of MK does not affect bFGF independent cell growth (data for human foreskin fibroblasts are shown on Figure 4). The presence of 10 ng/ml bFGF in the media results in small but significant increases in the numbers of fibroblasts (but not melanoma cells). This bFGF dependent effect is not affected by the presence of 0.75µM MK (Figure 4).

While not wanting to be limited by theory, the following is provided as a possible discussion of the above results.

MK, bFGF, and PF4 belong to different protein families and thus direct competition for binding to the high affinity bFGF receptor seems unlikely. However, MK, as well as PF4, may compete with bFGF for binding to the heparan sulfate moieties of low affinity bFGF receptors. As discussed hereinabove, recent findings indicate that binding of bFGF to low affinity receptors is necessary for creation of an "induced fit" conformation of bFGF able to bind to high affinity receptors (Yayon et al., 1991). Functional studies also indicate that intact low affinity receptors are necessary for bFGF-driven fibroblast growth and myoblast differentiation (Rapraeger et al., 1991). Thus, according to the above proposed model, the occupation of low affinity receptors by MK and PF4 is expected to decrease the number of bGFG molecules capable of binding to high affinity receptors.

The affinity of MK for cellular heparan sulfate proteoglycans is not known. Affinity of human PF4 for heparan sulfate proteoglycans of bovine endothelial cells is recently characterized (kd=2.87µM, Rybak et al., 1989). If MK and PF4 have similar affinities for heparin sulfate, then one might expect that micromolar of submicromolar concentrations of these proteins saturate low affinity receptors for bFGF. It is noteworthy, that, as judged by ED₅₀ recombinant MK is 2 to 5 fold more potent then human platelet-derived PF4 in inhibiting bFGF binding to high affinity BHK cell receptors.

Moreover, a comparison of the ED₅₀ obtained in mitogenic assays on human endothelial cells with recombinant PF4 (Maione et al., 1990, 1991) and recombinant MK demonstrates that the later protein is 5-10 fold more potent. This difference may be important in view of cytotoxicity of some heparin-binding proteins at high concentrations (Dauchel et al., 1989).

The results obtained with bovine ABAE cells indicate that high concentrations of MK (up to 0.75µM) inhibit cell growth but do not cause detachment of cells. In contrast, high concentrations of PF4 (above 0.3 µM) not only inhibit ABAE cell growth but also cause detachment of cells. Experiments with human HUVE cells indicate that the effects observed with high concentrations of MK may be counteracted by increasing the concentration of bFGF. It is important that MK affects only endothelial cells, but not human fibroblasts, human melanoma cells, rat PC12 cells and hamster BHK cells.

In view of these findings it is suggested that additional "cell-mediated" mechanisms of inhibition are involved in the anti-proliferatory activity of heparin-binding proteins directed toward endothelial cells. Inhibitory effects of MK have similar ED₅₀ values at different concentrations of bFGF suggesting that direct competition for receptors is not the sole factor. Similarly, Maione et al. (1991) found that with PF4 although a recombinant mutant of PF4 lacked affinity to heparin, it still retained potent angiostatic activity in vivo and inhibited proliferation of human endothelial cells in vitro.

### Deposit Of Biological Materials

E. coli strain M 1061 harboring pMKHC2 and E. coli strain BL2T LysS harboring pETMH2 have been deposited in the culture collections of American Cyanamid Company, Lederle Laboratories, Pearl River, New York, and with the American Type Culture Collection, 12301 Parklawn Drive, Rockville, MD, under accession number ATCC 68384, on August 13, 1990 and accession number ATCC 68401, on September 17, 1990, respectively and is available to the public pursuant to the appropriate legal standards for patents in the U.S. and other countries.

### Bibliography

1. Baird, A., and Bohlen, P. (1990) Fibroblast growth factors. In peptide growth factors and their receptors 1. (eds. Sporn, M.B., and Roberts, A.B.) pp369-418. Spring-Verlag, Berlin, Heidelberg.
2. Moscatelli, D., (1987). J. Cell Physiol 131:123-130.
3. Ruta, M., Howk, R., Ricca, G., Drohan, W., Zabelshansky, M., Laureys, G., Barton, D.E., Francke, U., Schlessinger, J., and Givol, D., (1988). Oncogene 3:9-15.
4. Coughlin, S.R., Barr, P.G., Cousens, L.S., Fretto, L.J., and Williams, P.T., (1988) kinase activity in vivo. J. Biol. Chem. 263:928-933.
5. Kornbluth, S., Paulson, K.S., and Hanafusa, H. (1988) Mol. Cell Biol. 8:5541-5544.
6. Lee, P.L., Johnson, D.E., Cousens, L.S., Fried, V.A., and Williams, L.T. (1989) Science 245: 57-60).
7. Pasquale, E.B., and Singer, S.J. (1989) Proc. Natl. Acad. Sci. USA 86:5449-5453.
8. Safran, A., Avivi, A., Orr-Urtereger, A., Neufeld, G., Lonai, P., Givol, D., and Yarden, Y. (1990) Oncogene 5:635-643.
9. Kiefer, M.C., Stephans, J.C., Crawford, K, Okino, K., and Barr, P.J. (1990) Proc. Natl. Acad. Sci. USA 87:6985-6989.
10. Vlodavsky, I., Folkman, J., Sullivan R., Freidman, R., Ishai-Michaeli, R. Sasse, and Klugsbrun, M. (1987) Proc. Natl. Acad. Sci. USA 84:2292-2296.
11. Moscatelli, D. (1988) J.Cell Biol. 107:753--759.
12. Bashkin, P., Doctrow, S., Svahn, C.M., Folkman, J., and Vlodavsky, I. (1989) Biochemistry 28,1737-1743.
13. Folkman, J., Klagsbrun, M., Sasse, J., Wadzinski, M., Ingber,D., and Vlodavsky, I. (1988) J. Pathol. 130:393-400.
14. Yayon, A., Klagsbrun, M., Esko, J.D., Leder, P., and Ornitz, D.M. (1991) Cell, 64:841-848.
15. Taylor, S., and Folkman, L. (1982) Nature 297:307-312.
16. Sato, Y., Abe, and Takaki, R. (1990) Biochem. Biophys.Res Commun. 172:595-600.
17. Sharpe, R.J., Byers, H.R., Scott, C.F., Bauer, S.I., and Maione, T.E. (1990) J.Natl. Cancer Inst. 82:848-853.
18. Maione, T.E., Gray, G.S., Petro, J. Hunt, A.T., Donner, A.L., Bauer, S.I., Carson, H.F., and Sharpe, R.G. (1990) Science (Washington, DC) 247:77-79.
19. Maione, T.E., Gray, G.S., Hunt, A.J., and Sharpe, R.J. (1991) Cancer Res. 51:2077-2083.
20. Kretschmer, P.J., Fairhurst, J.L., Decker, M.M., Chan, C.P. Gluzman, Y., Bohlen, P., and Kovesdi, I. (1991) Growth Factors 5:99-114.
21. Fafeur, V., Terman, B.I., Blum, J., and Bohlen, P. (1990) Growth Factors 3:237-245.
22. Jaffe, E.A. (1984) Culture and identification of large vessel endothelial cells. in Biology of Endothelial Cells. (ed. Jaffe, J.A.), Martinus-Nijhoff, New York.
23. Gospodarowicz. A., Cheng, J., Lui, G.M., D., Baird, and P. Bohlen (1984) Prox. Natl. Acad. Sci. USA 81:6963-6967.
24. Moscatelli, D.,Presta,M., Joseph-Silverstein, J., and Rifkin, D.B. (1986). J. Cell Physiol. 129: 273-276.
25. Hannan, R.L., Kourembanas, S., Flanders, K.C. Rogelj, S.J., Roberts, A.B., Faller, D.V., and Klagsbrun, M. (1987) Growth Factors 1:7-17.
26. Sato, Y., and Rifkin, D.B. (1988) J. Cell Biol. 107:1199-1205.
27. Maione, T.E., and Sharpe, R.J. (1990) TIBS 11:457-461.
28. Folkman, J., and Klagsbrun, M., (1987) Science (Washington DC) 235:442-447.
29. Seddon, A., Decker, M., Muller, T., Armellino, D., Kovesdi, I. Gluzman, Y., and Bohlen,P., (1991). Annals New York Acad. Sci., (in press).
30. Dauchel, M.C., Courty, J., Mereau, A., and Barritault, D., (1989). J. Cell. Physiol. 39:411-420.
31. Rapraeger. A.C., Krufka, A., and Olwin,B.B. (1991) Science (Washington, DC) 252:1705-1708.
32. Rybak, M.E., Gimrone, Jr., M.A., Davies, P.F., and Handin, R.I., (1989) Blood, 73:1534-1539.
33. Wanaka, A., Milbrandt, J., and Johnson, E.M., (1991) Development 111:455-468.
34. Gospodarowicz, D. (1975) Biol. Chem 250:2515-2520, 1975.
35. Leibroch et al. (1989) Nature; 341:149-153
36. Maisonpierre et al. (1990) Science 247:1446 -1451.
37. Lin et al. (1980) Science 246:1023-1025.
38. Mill et al. (1985) PNAS USA 82:7126-7130.
39. Schubert et al. (1990) Nature 344:868-870.
40. Berman et al. (1987) Cell 51:135-142.
41. Bohlen et al. (1984) PNAS USA 81:5364-5368.
42. Esch et al. (1985) PNAS USA 82:6507-6511).
43. Bohlen et al. (1985) Embo J. 4:1951-1956.
44. Gimenez - Gallego et al. (1985) Science 230:1385-1288.
45. Dickens and Peters (1984) Nature 326:833.
46. Delli Bovi et al. (1987) Cell 50:729-737.
47. Zhan et al. (1988) Cell Biol 8:3487-3495.
48. Marics et al. (1989) Oncogene 4:335-340.
49. Finch et al. (1989) Science 245:752-755.
50. Kadomatsu et al. Biochem (1988) Biophys Res Comm 151:1312-1318.
51. Rauvala (1989) EMBOJ 8:2933-2941.
52. Huber et al. (1990) Neurochem Res. 15:435-439.
53. Gautschi-Sova et al. (1986) Biochem. Biophys. Res. Comm. 140:1874-1880.
54. Aviv and Leder, (1972) PNAS USA 69:14088-14120).
55. Maniatis et al. (1982) Molecular Cloning. A laboratory Manual, Cold Spring Harbor Laboratory, Cold Spring Harbor, NY).
56. Sanger et al. (1988) PNAS USA 74:5463-5467.
57. Milner et al. (1989) Biochem Biophys. Res. Comm. 165:1096-1103.
58. Tomomura et al. (1990) J. Biol. Chem. 265:10765-10770).
59. Kadomatsu et al. (1990) J. Cell Biol. 110:607-616.
60. Von Heijne (1985) J. Mol. Biol. 184:99-105.
61. Von Heijne (1986) Nucl. Acids Res. 14:4683-4690.
62. Levi-Montalcini R. and Hamburger V. (1953) J. Exp. Zool. 123:233-278.

Sequence ID No.: 1

Sequence Type: Nucleic Acid

Molecule Type: DNA (genomic)

Sequence Length: 357 base pairs

Strandedness: Single

Topology: Linear

Original Source Organism: Mouse

Features: Coding Sequence from 1 to 357

AAA AAA AAA GAG AAG GTG AAG AAG GGC AGC GAG TGT TCG GAG TGG ACC 48
Lys Lys Lys Glu Lys Val Lys Lys Gly Ser Glu Cys Ser Glu Trp Thr
1               5                   10                  15

TGG GGG CCC TGC ACC CCC AGC AGC AAG GAC TGC GGC ATG GGC TTC CGC 96
Trp Gly Pro Cys Thr Pro Ser Ser Lys Asp Cys Gly Met Gly Phe Arg
            20                  25                  30

GAG GGT ACC TGT GGG GCC CAG ACC CAG CGC GTC CAT TGC AAG GTG CCC 144
Glu Gly Thr Cys Gly Ala Gln Thr Gln Arg Val His Cys Lys Val Pro
        35                  40                  45

TGC AAC TGG AAG AAG GAA TTT GGA GCC GAC TGC AAA TAC AAG TTT GAG 192
Cys Asn Trp Lys Lys Glu Phe Gly Ala Asp Cys Lys Tyr Lys Phe Glu
    50                  55                  60

AGC TGG GGG GCG TGT GAT GGG AGC ACT GGC ACC AAA GCC CGC CAA GGG 240
Ser Trp Gly Ala Cys Asp Gly Ser Thr Gly Thr Lys Ala Arg Gln Gly
65                  70                  75                  80

ACC CTG AAG AAG GCG CGG TAC ACT GCC CAG TGC CAG GAG ACC ATC CGC 288
Thr Leu Lys Lys Ala Arg Tyr Thr Ala Gln Cys Gln Glu Thr Ile Arg
                85                  90                  95

GTG ACT AAG CCC TGC ACC TCC AAG ACC AAG TCA AAG ACC AAA GCC AAG 336
Val Thr Lys Pro Cys Thr Ser Lys Thr Lys Ser Lys Thr Lys Ala Lys
            100                 105                 110

AAA GGA AAA GGA AAG GAC TA 357
Lys Gly Lys Gly Lys Asp
        115

Sequence ID No. 2:

Sequence Type: Amino Acid

Molecule Type: Protein

Sequence Length: 118 amino acids

Strandedness: Single

Topology: Linear

Note: This is the coding sequence from Sequence ID No. 1

Lys Lys Lys Glu Lys Val Lys Lys Gly Ser Glu Cys Ser Glu Trp Thr
1               5                   10                  15

Trp Gly Pro Cys Thr Pro Ser Ser Lys Asp Cys Gly Met Gly Phe Arg
            20                  25                  30

Glu Gly Thr Cys Gly Ala Gln Thr Gln Arg Val His Cys Lys Val Pro
        35                  40                  45

Cys Asn Trp Lys Lys Glu Phe Gly Ala Asp Cys Lys Tyr Lys Phe Glu
    50                  55                  60

Ser Trp Gly Ala Cys Asp Gly Ser Thr Gly Thr Lys Ala Arg Gln Gly
65                  70                  75                  80

Thr Leu Lys Lys Ala Arg Tyr Thr Ala Gln Cys Gln Glu Thr Ile Arg
                85                  90                  95

Val Thr Lys Pro Cys Thr Ser Lys Thr Lys Ser Lys Thr Lys Ala Lys
            100                 105                 110

Lys Gly Lys Gly Lys Asp
        115

Sequence ID No.: 3

Sequence Type: Nucleic Acid

Molecule Type: DNA (genomic)

Sequence Length: 366 base pairs

Strandedness: Single

Topology: Linear

Original Source Organism: Homo sapiens

Features: Coding Sequence from 1 to 366

AAA AAG AAA GAT AAG GTG AAG AAG GGC GGC CCG GGG AGC GAG TGC GCT 48
Lys Lys Lys Asp Lys Val Lys Lys Gly Gly Pro Gly Ser Glu Cys Ala
1               5                   10                  15

GAG TGG GCC TGG GGG CCC TGC ACC CCC AGC AGC AAG GAT TGC GGC GTG 96
Glu Trp Ala Trp Gly Pro Cys Thr Pro Ser Ser Lys Asp Cys Gly Val
            20                  25                  30

GGT TTC CGC GAG GGC ACC TGC GGG GCC CAG ACC CAG CGC ATC CGG TGC 144
Gly Phe Arg Glu Gly Thr Cys Gly Ala Gln Thr Gln Arg Ile Arg Cys
        35                  40                  45

AGG GTG CCC TGC AAC TGG AAG AAG GAG TTT GGA GCC GAC TGC AAG TAC 192
Arg Val Pro Cys Asn Trp Lys Lys Glu Phe Gly Ala Asp Cys Lys Tyr
    50                  55                  60

AAG TTT GAG AAC TGG GGT GCG TGT GAT GGG GGC ACA GGC ACC AAA GTC 240
Lys Phe Glu Asn Trp Gly Ala Cys Asp Gly Gly Thr Gly Thr Lys Val
65                  70                  75                  80

CGC CAA GGC ACC CTG AAG AAG GCG CGC TAC AAT GCT CAG TGC CAG GAG 288
Arg Gln Gly Thr Leu Lys Lys Ala Arg Tyr Asn Ala Gln Cys Gln Glu
                85                  90                  95

ACC ATC CGC GTC ACC AAG CCC TGC ACC CCC AAG ACC AAA GCA AAG GCC 336
Thr Ile Arg Val Thr Lys Pro Cys Thr Pro Lys Thr Lys Ala Lys Ala
            100                 105                 110

AAA GCC AAG AAA GGG AAG GGA AAG GAC TA 366
Lys Ala Lys Lys Gly Lys Gly Lys Asp
        115                 120

Sequence ID No. 4:

Sequence Type: Amino Acid

Molecule Type: Protein

Sequence Length: 121 amino acids

Strandedness: Single

Topology: Linear

Note: This is the coding sequence from Sequence ID No. 3

Lys Lys Lys Asp Lys Val Lys Lys Gly Gly Pro Gly Ser Glu Cys Ala
1               5                   10                  15

Glu Trp Ala Trp Gly Pro Cys Thr Pro Ser Ser Lys Asp Cys Gly Val
            20                  25                  30

Gly Phe Arg Glu Gly Thr Cys Gly Ala Gln Thr Gln Arg Ile Arg Cys
        35                  40                  45

Arg Val Pro Cys Asn Trp Lys Lys Glu Phe Gly Ala Asp Cys Lys Tyr
    50                  55                  60

Lys Phe Glu Asn Trp Gly Ala Cys Asp Gly Gly Thr Gly Thr Lys Val
65                  70                  75                  80

Arg Gln Gly Thr Leu Lys Lys Ala Arg Tyr Asn Ala Gln Cys Gln Glu
                85                  90                  95

Thr Ile Arg Val Thr Lys Pro Cys Thr Pro Lys Thr Lys Ala Lys Ala
            100                 105                 110

Lys Ala Lys Lys Gly Lys Gly Lys Asp
        115                 120

## Claims

1. The use of MK protein, alkylated MK protein, or a combination thereof for the manufacture of a composition useful for inhibiting cell growth.

2. The use of Claim 1, wherein the MK protein or alkylated MK protein is human MK protein, bovine MK protein, ovine MK protein, canine MK protein, porcine MK protein, feline MK protein, equine MK protein, avian MK protein, fish MK protein or the alkylated form thereof.

3. The use of MK protein, alkylated MK protein, or a combination thereof for the manufacture of a composition useful for controlling angiogenesis in an animal.

4. The use of Claim 3, wherein the MK protein or alkylated MK protein is human MK protein, bovine MK protein, ovine MK protein, canine MK protein, porcine MK protein, feline MK protein, equine MK protein, avian MK protein, fish MK protein or the alkylated form thereof.

5. The use of MK protein, alkylated MK protein, or a combination thereof, for the manufacture of a composition useful for controlling post-surgical bleeding in a warm-blooded animal.

6. The use of Claim 5, wherein the MK protein or alkylated MK protein is human MK protein, bovine MK protein, ovine MK protein, canine MK protein, porcine MK protein, feline MK protein, equine MK protein, avian MK protein, fish MK protein or the alkylated form thereof.

7. The use of MK protein, alkylated MK protein, or a combination thereof for the manufacture of a composition useful for inhibiting tumor growth in an animal.

8. The use of Claim 7, wherein the MK protein or alkylated MK protein is human MK protein, bovine MK protein, ovine MK protein, canine MK protein, porcine MK protein, feline MK protein, equine MK protein, avian MK protein, fish MK protein or the alkylated form thereof.

9. A method for screening in vitro for a cellular growth inhibitor, the method comprising: administering MK protein in a responsive cell assay.
